# EUROPEAN PATENT SPECIFICATION

(11) **EP 3 544 577 B1**
(45) Date of publication and mention of the grant of the patent: **09.12.2026**
(21) Application number: 17826111.1
(22) Date of filing: 18.12.2017
(51) Int. Cl.: A61K 8/39, A61Q 15/00, A61Q 19/00, A61K 8/86, A61Q 19/10

(54) **PEARLING AGENT AND PEARLESCENT COMPOSITIONS**
PERLMITTEL UND PERLMUTTARTIGE ZUSAMMENSETZUNGEN
AGENT NACRANT ET COMPOSITIONS NACRÉES

(30) Priority: 27.12.2016 US 201662439223 P
(43) Date of publication of application: 02.10.2019
(73) Proprietor: Colgate-Palmolive Company, New York, NY 10022 (US)
(72) Inventor: DENNIS, Mavis, Sayreville, New Jersey 08872 (US); KOZUBAL, Cheryl, Somerset, New Jersey 08873 (US); KENNEDY, Sharon, Randallstown, MD 21133 (US)
(74) Representative: Wichmann, Hendrik
(86) International application number: PCT/US2017/067119
(87) International publication number: WO 2018/125645

(56) References cited:
- EP-A1- 3 000 459
- EP-B1- 3 000 459
- WO-A1-2004/112739
- WO-A1-2015/112787
- WO-A1-2016/109517
- DE-A1- 19 511 572
- US-A- 4 767 617
- US-A1- 2008 206 155

## Description

### BACKGROUND

Pearling agents or pearlants are used to impart a pearlescent or pearlescence-like appearance to cosmetic and personal care compositions, such as soaps, deodorants, shampoos, and body gels. Various known substances have typically been used to achieve pearlescent or iridescent effects, for example mica powders, fish scales, basic lead carbonate, bismuth oxychloride, and titanium dioxide pigments.

However, conventional personal care compositions often include a large number of ingredients working together to create a stable composition with the desired functional characteristics. The complexity of such compositions require extensive amounts of time and logistical efforts to assure the effectiveness and compatibility of the various ingredients when used together.

Accordingly, there is a desire for pearling agents which are compatible with other ingredients in cosmetic or personal care compositions, and that allow for the formulation of cosmetic or personal care compositions that have a minimum number of ingredients while still maintaining desired levels of stability, efficiency, and aesthetic characteristics. EP 3 000 459 A1 discloses an antiperspirant composition comprising at least 3.5 % by weight of zinc phenolsulphonate or zinc lactate, wherein said composition is substantially free of aluminum salts. WO 2016/109517 A1 discloses cosmetic emulsions are disclosed that can remain stable even with relatively high salt based cosmetic astringent content, wherein, in one aspect, an emulsion can include a water phase including water and a rheology modifier including microcrystalline cellulose and cellulose gum. US 2008/0206155 A1 discloses a stable non-alcoholic foamable pharmaceutical emulsion composition that includes an unctuous emollient, at a concentration of about 0.5% to about 49% by weight; at least one multi-active agent; at a concentration of about 0.5% to about 15% by weight; water; an effective amount of an active pharmaceutical agent having a degree of solubility in the emulsion composition; and at least one liquefied or compressed gas propellant at a concentration of about 3% to about 25% by weight of the total composition. DE 19511572 A1 discloses low-viscosity opacifying agent concentrates based on wax bodies having a solids content ranging from 40 to 60 wt %. The concentrates contain as emulsifiers at least one hydrophilic non-ionic surfactant with an HLB value of more than 10 and at least one hydrophobic non-ionic surfactant with an HLB value of less than 10.

### BRIEF SUMMARY

This section is intended merely to introduce a simplified summary of some aspects of one or more implementations of the present disclosure. Further areas of applicability of the present invention will become apparent from the detailed description provided hereinafter. This summary is not an extensive overview, nor is it intended to identify key or critical elements of the present teachings, nor to delineate the scope of the disclosure. Rather, its purpose is merely to present one or more concepts in simplified form as a prelude to the detailed description below.

The invention refers to the use of a pearling agent as defined in the claims.

The surfactant combination may consist essentially of Steareth-2 and Steareth-21.

### DETAILED DESCRIPTION

Reference will now be made in detail to the various implementations in the present disclosure, examples of which may be illustrated in any accompanying drawings and figures. The examples are described below to provide a more complete understanding of the components, processes, compositions, and apparatuses disclosed herein. Any examples given are intended to be illustrative, and not restrictive. However, it will be apparent to one of ordinary skill in the art that the invention may be practiced without these specific details. In other instances, well-known methods, procedures, and components have not been described in detail so as not to unnecessarily obscure aspects of the disclosure.

Throughout the specification and claims, the following terms take the meanings explicitly associated herein, unless the context clearly dictates otherwise. The phrases "in some examples" and "in an example" as used herein do not necessarily refer to the same example(s), though they may. Furthermore, the phrases "in another example" and "in some other examples" as used herein do not necessarily refer to a different example, although they may.

As used herein, the term "or" is an inclusive operator, and is equivalent to the term "and/or," unless the context clearly dictates otherwise. The term "based on" is not exclusive and allows for being based on additional factors not described, unless the context clearly dictates otherwise. In the specification, the recitation of "at least one of A, B, and C," includes examples containing A, B, or C, multiple examples of A, B, or C, or combinations of A/B, A/C, B/C, A/B/B/ B/B/C, A/B/C, etc. In addition, throughout the specification, the meaning of "a," "an," and "the" include plural references. The meaning of "in" includes "in" and "on."

It will also be understood that, although the terms first, second, etc. may be used herein to describe various elements, these elements should not be limited by these terms. These terms are only used to distinguish one element from another. For example, a first object, component, or step could be termed a second object, component, or step, and, similarly, a second object, component, or step could be termed a first object, component, or step, without departing from the scope of the invention. The first object, component, or step, and the second object, component, or step, are both, objects, component, or steps, respectively, but they are not to be considered the same object, component, or step. It will be further understood that the terms "includes," "including," "comprises" and/or "comprising," when used in this specification, specify the presence of stated features, steps, operations, elements, and/or components, but do not preclude the presence or addition of one or more other features, steps, operations, elements, components, and/or groups thereof. Further, as used herein, the term "if" may be construed to mean "when" or "upon" or "in response to determining" or "in response to detecting," depending on the context.

All physical properties that are defined hereinafter are measured at 20° to 25° Celsius unless otherwise specified.

When referring to any numerical range of values herein, such ranges are understood to include each and every number and/or fraction between the stated range minimum and maximum, as well as the endpoints. For example, a range of 0.5-6% would expressly include all intermediate values of, for example, 0.6%, 0.7%, and 0.9%, all the way up to and including 5.95%, 5.97%, and 5.99%, among many others. The same applies to each other numerical property and/or elemental range set forth herein, unless the context clearly dictates otherwise.

Additionally, all numerical values are "about" or "approximately" the indicated value, and take into account experimental error and variations that would be expected by a person having ordinary skill in the art. It should be appreciated that all numerical values and ranges disclosed herein are approximate values and ranges, whether "about" is used in conjunction therewith.

Unless otherwise specified, all percentages and amounts expressed herein and elsewhere in the specification should be understood to refer to percentages by weight. The amounts given are based on the active weight of the material.

With regard to procedures, methods, techniques, and workflows that are in accordance with the disclosure, some operations in the procedures, methods, techniques, and workflows disclosed herein may be combined and/or the order of some operations may be changed.

As used herein, the term "pearling agent" or "pearlant" refers to a substance used to impart, emulate, or recreate the aesthetic appearances of pearl in a composition, such as a personal care composition. For example, pearling agents are associated with producing a pearlescent texture or visual effect, brilliant colors, and luster. In some cases, pearling agents reflect and refract light to create a pearl-like luster and/or exhibit interference color effects and iridescence.

In some cases, the pearling agent is selected for its compatibility with the other ingredients in a personal care composition, or is selected for its ability to provide additional qualities to personal care composition in addition to pearlescence. For example, the pearling agent may be selected to enable the solubilization of other ingredients in the personal care composition or to act as a surfactant. In other examples, the pearling agent may also help resist degradation of the composition in a low pH environment.

The invention refers to the use of a pearling agent to impart a pearlescent or pearlescence-like appearance to cosmetic and personal care compositions, wherein the pearling agent is a surfactant combination of one or more low HLB surfactants having an HLB value from 3 to 6, in an amount from 2 weight % to 5 weight % based on the total weight of the cosmetic or personal care composition; and one or more high HLB surfactants having an HLB value from 12 to 16, in an amount from 1.5 weight % to 4.5 weight % based on the total weight of the cosmetic or personal care composition. For example, the inventors have surprisingly discovered that a system of surfactants having both high and low hydrophilic-lipophilic balances (HLB) allows for the creation of personal care compositions with a pearlescent appearance while maintaining compatibility with other ingredients of the personal care compositions, which are selected to impart desired stability, efficacy, and aesthetic characteristics. In other examples, the pearling agent imparts a pearlescent appearance to the personal care composition while simultaneously serving as a surfactant.

The pearling agent includes a combination of one or more low HLB surfactants having a low HLB value and one or more high HLB surfactants having a high HLB value as defined in the claims. In some examples, this surfactant combination is the only pearling agent in the personal care composition.

The one or more low HLB surfactants according to the claims has an HLB value from 3 to 6 or an HLB value of 5. For example, the one or more low HLB surfactants may include at least one of Steareth-2, Steareth-4, Steareth-6, Steareth-7, Ceteareth-2, Ceteareth-3, Ceteareth-4, and Ceteareth-5. In other instances, the one or more low HLB surfactants consist of only one of Steareth-2, Steareth-4, Steareth-6, Steareth-7, Ceteareth-2, Ceteareth-3, Ceteareth-4, or Ceteareth-5 or consists essentially of only one of Steareth-2, Steareth-4, Steareth-6, Steareth-7, Ceteareth-2, Ceteareth-3, Ceteareth-4, or Ceteareth-5. In one example of such an embodiment, the composition contains one low HLB surfactant, where the low HLB surfactant consists of at least 99% of only one of Steareth-2, Steareth-4, Steareth-6, Steareth-7, Ceteareth-2, Ceteareth-3, Ceteareth-4, or Ceteareth-5. In one embodiment, the low HLB surfactant consists essentially of Steareth-2.

The one or more high HLB surfactants according to the claims has an HLB value from 12 to 16 or an HLB value of 15. For example, the one or more high HLB surfactants may include Steareth-10, Steareth-11, Steareth-13, Steareth-15, Steareth-20, Steareth-21, Ceteareth-6, Ceteareth-7, Ceteareth-8, Ceteareth-9, Ceteareth-10, Ceteareth-11, and Ceteareth-12. In other instances, the one or more low HLB surfactants may consist only of one of Steareth-10, Steareth-11, Steareth-13, Steareth-15, Steareth-20, Steareth-21, Ceteareth-6, Ceteareth-7, Ceteareth-8, Ceteareth-9, Ceteareth-10, Ceteareth-11, or Ceteareth-12 or may consist essentially of only one of Steareth-10, Steareth-11, Steareth-13, Steareth-15, Steareth-20, Steareth-21, Ceteareth-6, Ceteareth-7, Ceteareth-8, Ceteareth-9, Ceteareth-10, Ceteareth-11, or Ceteareth-12. In one such example, the pearling agent contains one high HLB surfactant, and the high HLB surfactant consists of at least 99% of only one of Steareth-10, Steareth-11, Steareth-13, Steareth-15, Steareth-20, Steareth-21 Ceteareth-6, Ceteareth-7, Ceteareth-8, Ceteareth-9, Ceteareth-10, Ceteareth-11, or Ceteareth-12. In another example, the pearling agent contains one high HLB surfactant, and the high HLB surfactant consists essentially of Steareth-21.

The one or more low HLB surfactants have an HLB value from 3 to 6 and the one or more high HLB surfactants have an HLB value from 12 to 16. For example, the one or more low HLB surfactants may have an HLB value of 5 and the one or more high HLB surfactants may have an **HLB** value of 15. The one or more surfactants may include Steareth-2 and Steareth-21. In various instances, the one or more surfactants may consist essentially of Steareth-2 and Steareth-21. In some instances, the one or more surfactants may consist of at least 99% Steareth-2 and Steareth-21.

The pearling agent is used to impart a pearlescent or pearlescence-like appearance to cosmetic and personal care compositions. The personal care composition may include a combination of surfactants or may include only the pearling agents as surfactants, and the amount of surfactant in the composition may depend on the type of personal care composition.

**In** some examples, the personal care composition is a body gel and the personal care composition may include from 4 weight % to 15 weight % surfactant, from 7 weight % to 12 weight % surfactant, or from 8 weight % to 10 weight % surfactant, based on the total weight of the personal care composition.

**In** other examples, the personal care composition is an antiperspirant and/or deodorant composition and the personal care composition may include from 1 weight % to 7 weight % surfactant or from 3 weight % to 6 weight % surfactant, based on the total weight of the personal care composition.

**In** some cases, the surfactant will consists essentially of the pearling agent. In other cases, the personal care composition will include additional surfactants in addition to the pearling agent. As defined in the claims, the amount of low HLB and high HLB surfactants is based on the total weight of the cosmetic or personal care composition. In addition, in cases where the personal care composition includes a combination of surfactants, the amount of pearling agent may additionally be established in terms of the total amount of surfactants in the composition. For example, the personal care composition may include from 3% to 15% pearling agent, based on the total weight of surfactants in the personal care composition. In other instances, the personal care composition may include from 4% to 10% pearling agent, based on the total weight of surfactants in the personal care composition.
The personal care composition includes from 2 weight % to 5 weight % low HLB surfactants and from 1.5 weight % to 4.5 weight % high HLB surfactants.

### EXAMPLES

Aspects of the present disclosure may be further understood by referring to the following examples. The examples are illustrative, and are not intended to be limiting. Tables 1 and 2 illustrate personal care compositions according to examples of the present disclosure.

As illustrated in Tables 1 and 2, the personal care compositions may include additional ingredients to provide functional and aesthetic characteristics to the personal care composition, such as carriers, antiperspirant or deodorant actives, surfactants and/or emulsifiers, preservatives, pH modifiers, emollients, and fragrances. However, the inclusion of surfactants according to the examples and embodiments of this disclosure, as described above, provide the personal care compositions with a pearlescent appearance.

For example, as illustrated in Table 1, an antiperspirant and/or deodorant composition may include up to five types of ingredients: antiperspirant and/or deodorant actives, carriers, surfactants, and preservatives. In other examples, the antiperspirant and/or deodorant composition may also include optional ingredients, such as fragrances or emollients. In other examples, in order to minimize the number of ingredients and provide a streamlined, low-cost, composition, the antiperspirant and/or deodorant composition may be limited to only the antiperspirant and/or deodorant actives, the carriers, the surfactants, the preservatives, and optionally, the fragrances and/or emollients. The use of a surfactant including a combination of low HLB and high HLB surfactants, as described above, provides the antiperspirant and/or deodorant composition with a pearlescent appearance.

As illustrated in Table 2, a shower gel composition may include up to five types of ingredients: carriers, surfactants and/or emulsifiers, preservatives, and pH modifiers. In other examples, the shower gel composition may also include optional ingredients, such as fragrances or emollients. In other examples, in order to minimize the number of ingredients and provide a streamlined, low-cost, composition, the shower gel composition may be limited to only the carriers, surfactants and/or emulsifiers, preservatives, and pH modifiers, and optionally, the fragrances and/or emollients. The use of a surfactant including a combination of low HLB and high HLB surfactants as described above provides the shower gel composition with a pearlescent appearance.

The personal care composition may include one or more carriers or solvents. For example, the personal care composition may be provided as an oil-in-water emulsion using primarily water as the carrier. The carrier may also include other co-solvents which are miscible with water. However, in various preferred instances, the carrier consists only of water or consists essentially of water, such as a carrier that consists of at least 99% water.

The personal care composition also includes one or more antiperspirant or deodorant actives. The antiperspirant or deodorant actives are selected to be compatible with the other ingredients in the personal care composition. For example, the antiperspirant or deodorant actives may be selected to maintain the overall pH of the personal care composition in a range from about 3 to about 5. In other instances, the antiperspirant or deodorant actives are selected to be dissolvable in the carrier.

In one example, the antiperspirant or deodorant actives includes at least one of aluminum chlorohydrate, aluminium chloride, and aluminum zirconium. In other examples, the antiperspirant or deodorant actives include potassium aluminum sulfate or potassium aluminum sulfate crystals. The deodorant actives may also include at least one of alum, potassium aluminum sulfate, zinc oxide, ethylhexylglycerin, octenidine hydrochloride, zinc citrate, zinc pyrithione, silver compounds. The antiperspirant or deodorant actives may also consist or consist essentially of aluminum chlorohydrate, aluminium chloride, and aluminum zirconium, potassium aluminum sulfate, or potassium aluminum sulfate crystals. In some cases, the antiperspirant or deodorant actives are the only active antiperspirant ingredient or the only active odor control ingredient in the personal care composition.

The personal care composition may include one or more preservatives to improve an antimicrobial characteristic of the personal care composition or to improve a storage life and/or prevent microbial contamination.

The preservatives may also enhance the functional characteristics of the personal care composition. For example, the preservative may provide additional deodorant or emollient effects to the personal care composition.

The one or more preservatives may include caprylyl glycol, phenoxyethanol, ethylhexylglycerin, citric acid, benzoic acid, lactic acid, sodium benzoate, or sodium salicylate, or may consist essentially of caprylyl glycol, phenoxyethanol, ethylhexylglycerin, citric acid, benzoic acid, lactic acid, sodium benzoate, or sodium salicylate, with only trace amounts of other materials. In other examples, the preservatives may include one or more of benzoic acid, phenoxyethanol, citric acid, lactic acid, sodium salicylate, benzalkonium chloride, benzethonium chloride, 5-bromo-5-nitro-1,3-dioxane, 2-bromo-2-nitropropane-1,3-diol, alkyl trimethyl ammonium bromide, N-(hydroxymethyl)-N-(1,3-dihydroxy methyl-2,5-dioxo-4-imidaxolidinyl-N-(hydroxy methyl)urea, 1-3-dimethyol-5,5-dimethyl hydantoin, formaldehyde; iodopropynl butyl carbamate, butyl paraben, ethyl paraben, methyl paraben, propyl paraben, methyl isothiazolinone, methyl-chloroisothiazoline, phenoxyethanol, tris-hydroxyethyl-hexahydrotriazine, methylisothiazolinone, 5-chloro-2-methyl-4-isothiazolin-3-one, 1,2-dibromo-2,4-dicyanobutane, 1-(3-chloroalkyl)-3,5,7-triaza-azoniaadam-antane chloride, and organic acids or combinations thereof. In such examples, the preservatives in the personal care composition may consist of at least 99% preservative, or may consist essentially of caprylyl glycol.

The personal care composition may also include one or more pH modifying agents. PH modifying agents among those useful herein include acidifying agents to lower pH, basifying agents to raise pH and buffering agents to control pH within a desired range. For example, the personal care composition may include sodium lactate, lactic acid, citric acid, hydrochloric acid, glycolic acid, sodium hydroxide, potassium chloride, monosodium citrate, disodium citrate, monosodium malate, sodium carbonate, bicarbonates, sesquicarbonates, borates, silicates, monosodium phosphate, trisodium phosphate, pyrophosphate salts, imidazole, as pH modifying agents.

The personal care composition includes one or more surfactants or emulsifiers. In some cases, the pearling agent also acts as a surfactant. In other examples, the personal care composition includes surfactants or emulsifiers other than or in addition to the pearling agent.

In various examples, the surfactants are selected to provide an adequate or desired viscosity to the personal care composition. In other examples, the surfactants are selected to enable the solubilization of other ingredients in the personal care composition. In yet other cases, the surfactants are selected to resist degradation of the composition in a low pH environment.

A combination of surfactants may be used. Suitable anionic surfactants include without limitation water-soluble salts of C₈₋₂₀ alkyl sulfates, sodium laureth sulphates, sulfonated monoglycerides of C₈₋₂₀ fatty acids, sarcosinates, taurates and the like. Illustrative examples of these and other classes include sodium lauryl sulfate, sodium cocoyl monoglyceride sulfonate, sodium lauryl sarcosinate, sodium lauryl isoethionate, sodium laureth carboxylate, and sodium dodecyl benzenesulfonate. Suitable nonionic surfactants include without limitation poloxamers, polyoxyethylene sorbitan esters, fatty alcohol ethoxylates, alkylphenol ethoxylates, tertiary amine oxides, tertiary phosphine oxides, dialkyl sulfoxides and the like. Suitable amphoteric surfactants include, without limitation, derivatives of C₈₋₂₀ aliphatic secondary and tertiary amines having an anionic group such as carboxylate, sulfate, sulfonate, phosphate or phosphonate. A suitable example is cocamidopropyl betaine or coco glucoside.

The personal care composition may also include a surfactant system including a combination of an anionic surfactant and a zwitterionic surfactant. For example, the surfactant system may include 4 weight % to 15 weight %, 7 weight % to 12 weight %, 8 weight % to 11 weight %, or 10 weight % surfactant system, based on the total weight of the personal care composition.

Suitable anionic surfactants may include, but are not limited to, those surface-active or detergent compounds that contain an organic hydrophobic group containing generally 8 to 26 carbon atoms or generally 10 to 18 carbon atoms in their molecular structure and at least one water-solubilizing group selected from sulfonate, sulfate, and carboxylate so as to form a water-soluble detergent. Usually, the hydrophobic group will comprise a C₈-C₂₂ alkyl, or acyl group. Such surfactants are employed in the form of water-soluble salts and the salt-forming cation usually is selected from sodium, potassium, ammonium, magnesium and mono-, di- or tri-C₂-C₃ alkanolammonium, with the sodium, magnesium and ammonium cations again being the usual ones chosen.

Some examples of suitable anionic surfactants include, but are not limited to alkyl ether sulfates, for example linear C₈-C₁₈ alkyl ether sulfates, and salts thereof, preferably the sodium, potassium, ammonium, and ethanolammonium salts thereof.

Suitable anionic ether sulfates have the formula R(OC₂H₄)ₙ OSO₃M wherein n is 1 to 12, or 1 to 5, and R is an alkyl, alkylaryl, acyl, or alkenyl group having 8 to 18 carbon atoms, for example, an alkyl group of C₁₂-C₁₄ or C₁₂-C₁₆, and M is a solubilizing cation selected from sodium, potassium, ammonium, magnesium and mono-, di- and triethanol ammonium ions. Exemplary alkyl ether sulfates contain 12 to 15 carbon atoms in the alkyl groups thereof, e.g., sodium laureth (2 EO) sulfate. Some preferred exemplary anionic surfactants that may be used include sodium laurel ether sulfate (SLES), sodium lauryl sulfate, and ammonium lauryl sulfate.

The personal care compositions may include a single anionic surfactant. In some embodiments, the anionic surfactant may be present in an amount of 0.01 % to 20 % anionic surfactant based on the total weight of the personal care composition. In other examples, the personal care composition may include 0.1 weight % to 10 weight %, 3 weight % to 10 weight %, 2 weight % to 8 weight %, 3 weight % to 8 weight %, 4 weight % to 9 weight %, or 5 weight % to 7 weight % anionic surfactant.

The personal care composition may also include a zwitterionic surfactant. Suitable zwitterionic surfactants include betaines and sultaines. In some embodiments, the zwitterionic surfactant includes a betaine having a quaternary ammonium or phosphonium ion as the cationic group and a carboxylate group as the anionic group, or comprises a betaine having a quaternary ammonium ion as the cationic group and a carboxylate group as the anionic group (i.e., a quaternary ammonium carboxylate betaine). Typical alkyldimethyl betaines include, but are not limited to, decyl dimethyl betaine or 2-(N-decyl-N, N-dimethylammonia)acetate, coco dimethyl betaine or 2-(N-coco N, N-dimethylammonia)acetate, myristyl dimethyl betaine, palmityl dimethyl betaine, lauryl dimethyl betaine, cetyl dimethyl betaine, stearyl dimethyl betaine, etc. The amidobetaines similarly include, but are not limited to, cocoamidoethylbetaine, cocoamidopropyl betaine and the like. The betaine may be coco (C₈-C₁₈) amidopropyl dimethyl betaine. Two examples of betaine surfactants that can be used are EMPIGEN^{™} BS/CA from Huntsman, and Tegobetaine F50 from BASF. Other suitable zwitterionic surfactants include amine oxides.

The personal care compositions may include a single zwitterionic surfactant. The zwitterionic surfactant may be present in an amount of 0.01 % to 10 %, based on a total weight of the personal care composition. In other examples, the personal care composition may include 1 weight % to 9 weight %, 1 weight % to 5 weight %, 3 weight % to 5 weight %, 4 weight % to 12 weight %, 2.5 weight % to 5 weight %, or 3 weight % to 4 weight % zwitterionic surfactant.

In some embodiments, the surfactant system includes anionic surfactant and zwitterionic surfactant in a weight ratio of about 1:3 to about 3:1. In some examples, the ratio of anionic: zwitterionic surfactant is about 2:1 to about 1:2, about 1:1 to about 2.3:1, about 1.2:1 to about 2.1:1, about 1.4:1 to about 2.1:1, about 1.5:1 to about 2:1; about 1.6:1 to about 1.8:1, or about 1.7:1, of the total composition by weight; for example about 1:1, about 1.1:1, about 1.2:1, about 1.3:1, about 1.4:1, about 1.5:1, about 1.6:1, about 1.7:1, about 1.8:1, about 1.9:1, about 2:1, about 2.1:1, about 2.2:1, about 2.3:1, about 2.4:1, or about 2.5:1.

As described above, the pearling agent may also act as a surfactant. In such cases, the surfactant may also include combinations of Steareth-2, Steareth-4, Steareth-6, and Steareth-7, and Steareth-10, Steareth-11, Steareth-13, Steareth-15, Steareth-20, and Steareth-21.

The personal care composition may optionally include one or more emollients and/or fragrances. For example, the personal care composition may include non-volatile emollients in any desired amount to achieve a desired emollient effect. Similarly, the personal care composition may optionally include one or more fragrances as a non-essential ingredient. A variety of fragrances can be used in the personal care composition if a scented product is desired.

Generally, viscosity is a characteristic that is considered when creating personal care compositions, and especially roll-on formulations. For example, when the viscosity of a roll-on antiperspirant and/or deodorant composition is too low, it may be too runny and it may leak from the roll-on dispenser during storage or use. In some cases, this will not only affect the delivery and aesthetics of the antiperspirant and/or deodorant composition but may also negatively affect the homogeneity of the ingredients in the antiperspirant and/or deodorant composition or personal care composition. On the other hand, if the viscosity of the antiperspirant and/or deodorant composition is too high, packaging into and delivery through a roll-on dispenser will be difficult.

Examples of rheology modifiers that can be used to modify the viscosity of a personal care composition that are useful for understanding the present invention include inorganic salts, isopropyl palmitate, isopropyl myristate, a polymer, salts or other electrolytes such as e.g., sodium chloride, and other mono-, di- and trivalent salts, and a hydrotrope. The rheology modifier may also include a brine solution of sodium chloride. In some examples, one or more rheology modifiers may be present in a total amount of from 0.01 % to 10%, based on a total weight of the personal care composition. For example, personal care compositions useful for understanding the invention may include from 0.1 weight % to 8 weight %, or from about 0.01 weight % to about 6 weight % rheology modifiers.

The personal care composition may achieve a desirable viscosity using only the ingredients described herein and may not require added rheology modifiers to achieve a target viscosity. For example, the personal care composition does not include thickeners or viscosity modifiers, such as hydroxyethyl cellulose, modified hydroxyethyl cellulose, and scleroglucan. The personal care composition does not include gelling agents, such as stearyl alcohol, dibenzylidene sorbitol, and polyethylene glycol.

Accordingly, it is desirable to select ingredients, and their specific amounts, for personal care compositions that achieve an advantageous range of viscosity to ensure product manufacturability, stability, and quality, as well as adequate or satisfactory delivery through consumer dispensers.

Depending on the type of personal care composition, the viscosity may be from 900 mPas to 15,000 mPas at 25° C. For example, roll-on type antiperspirant and/or deodorant compositions may have a viscosity from 900 mPas to 4,000 mPas at 25° C. Similarly, shower-gel type personal care compositions may have a viscosity from 2,000 mPas to 15,000 mPas at 25° C. In other embodiments, the viscosity of the personal care composition may be from 3,000 mPas to 9,000 mPas at 25° C. Viscosity is measured in mPas by using a Brookfield Viscometer at 25° C with spindle 4 or 5 at an RPM setting of 20.

Tables 1-3 illustrate pearlescent deodorant and/or antiperspirant compositions according to the present disclosure. As illustrated in Tables 1-3, the pearling agent is used as the surfactant in the deodorant and/or antiperspirant compositions and provides a pearlescent appearance.

**Table 1**

| **Composition 1** | | |
|---|---|---|
| **Ingredients** | | **Weight %** |
| Aluminium Chlorohydrate | Antiperspirant Active | 8.0 % |
| Steareth -2 | Surfactant / Pearling Agent | 3.5 % |
| Steareth -21 | Surfactant / Pearling Agent | 2.5 % |
| Caprylyl Glycol | Preservative | 0.5 % |
| Water | Carrier | Q. S. |
| **Total** | | **100 %** |

**Table 2**

| **Composition 2** | | |
|---|---|---|
| **Ingredients** | | **Weight %** |
| Aluminium Chlorohydrate | Antiperspirant Active | 12.0 % |
| Steareth -2 | Surfactant / Pearling Agent | 3.5 % |
| Steareth -21 | Surfactant / Pearling Agent | 2.5 % |
| Caprylyl Glycol | Preservative | 0.5 % |
| Water | Carrier | Q. S. |
| **Total** | | **100** % |

**Table 3**

| **Composition 3** | | |
|---|---|---|
| **Ingredients** | | **Weight %** |
| Potassium Aluminum Sulphate | Deodorant Active | 2.0 % |
| Steareth -2 | Surfactant / Pearling Agent | 3.5 % |
| Steareth -21 | Surfactant / Pearling Agent | 2.5 % |
| Caprylyl Glycol | Preservative | 0.5 % |
| Water | Carrier | Q. S. |
| **Total** | | **100 %** |

The deodorant and/or antiperspirant composition of Tables 1 and 2 were prepared as follows: the formula amounts of the surfactants were mixed together and heated to 80 °C. The formula amounts of water and preservative were mixed together and heated to 80 °C. The resulting mixture was then added to the surfactant mixture, homogenized for 3 minutes, and allowed to cool to 65 °C. The formula amounts of antiperspirant active was then added to the mixture and allowed to cool to room temperature.

The deodorant and/or antiperspirant composition of Table 3 was prepared as follows: the formula amounts of the surfactants were mixed together and heated to 80 °C. The formula amounts of water and preservative were mixed together and heated to 80 °C. The formula amounts of deodorant active was added to the mixture of water and preservative, and the resulting mixture was added to the surfactant mixture. The complete mixture was homogenized for 3 minutes and allowed to cool to room temperature.

All of the deodorant and/or antiperspirant compositions of Tables 1-3 displayed comparable stability, irritability, and viscosity characteristics to those of commercial counterparts. In addition, a visual evaluation of the deodorant and/or antiperspirant compositions of Tables 1-3 indicated they all possessed a recognizable pearlescent appearance due to the combination of high HLB and low HLB surfactants used as the pearling agent.

For example, the antiperspirant and/or deodorant compositions of Tables 1-3 were analyzed for their micro-robustness using theF APET (antimicrobial preservation effectiveness test), which measures the ability of a formulation to resist the growth of yeast, mold, and bacteria.

The APET test is a familiar test used in the cosmetic and personal care industry. The APET test is conducted under either aged or unaged conditions. Aged means that the product being tested has been maintained at a temperature of 40 °C and 75% relative humidity for 8 weeks in final packaging, whereas the unaged test is performed on fresh product after manufacture. The test measures the growth of an inoculum of bacteria or mold in the presence of the tested composition. APET results in either a pass or fail rating. A passing APET test requires a greater than 99.9% reduction in the level of bacteria in the inoculum and a greater than 90.0% reduction in the level of mold in the inoculum after 7 days.

As illustrated in Table 4, the antiperspirant and/or deodorant compositions of Tables 1-3 meet APET acceptance criteria.

**Table 4**

| | **Composition 1** | **Composition 2** | **Composition 3** |
|---|---|---|---|
| | **Sample** meet APET acceptance criteria | **Sample** meet APET acceptance criteria | **Sample** meet APET acceptance criteria |
| APET | Bacteria: > 99.9% reduction | Bacteria: > 99.9% reduction | Bacteria: > 99.9% reduction |
| | Mold :> 90.0% reduction | Mold :> 90.0% reduction | Mold :> 90.0% reduction |

The antiperspirant and/or deodorant compositions of Tables 1-3 were also analyzed for stability in terms of odor, color, and appearance before and after aging, after exposure to sunlight, and after repeated freeze/thaw cycles. **In** particular, samples of the antiperspirant and/or deodorant compositions were aged for up to 13 weeks, which is the extent of time commonly used to predict a 24 month shelf life under normal conditions. The compositions of Tables 1-3 were stored at different temperatures and relative humidity levels for up to 13 weeks. **In** addition, the compositions of Tables 1-3 were exposed to natural sunlight for 4 weeks and subjected to repeated freeze/thaw cycles for 7 days.

Odor, color, and appearance was evaluated by a trained stability evaluator comparing a reference sample stored at cold temperature for each composition to samples of the respective compositions at different time and temperature points, after prolonged exposure to sunlight, and after 7 days of freeze/thaw cycles. Odor, color, and appearance was rated as acceptable based on the odor, color, and appearance of the reference sample.

As illustrated in Tables 5-7 below, the compositions of Tables 1-3 displayed no spoilage or significant degradation in color, odor, or appearance.

**Table 5**

| **Stability Data - Composition 1** | | | | |
|---|---|---|---|---|
| Conditions | Time points | **Color** | **Odor** | **Appearance** |
| 25 °C / 60% RH | 4 weeks | Acceptable | Acceptable | Acceptable |
| | 8 weeks | | | |
| | 13 weeks | | | |
| 40 °C /75% RH | 4 weeks | Acceptable | Acceptable | Acceptable |
| | 8 weeks | | | |
| | 13 weeks | | | |
| 4 °C | 4 weeks | Acceptable | Acceptable | Acceptable |
| | 8 weeks | | | |
| | 13 weeks | | | |
| Natural Sunlight / UV | 4 weeks | Acceptable | Acceptable | Acceptable |
| Freeze/Thaw (-10 °C, -30 °C, and room temperature) | 7 days | Acceptable | Acceptable | Acceptable |

**Table 6**

| **Stability Data** - **Composition 2** | | | | |
|---|---|---|---|---|
| Conditions | Time points | **Color** | **Odor** | **Appearance** |
| 25 °C / 60% RH | 4 weeks | Acceptable | Acceptable | Acceptable |
| | 8 weeks | | | |
| | 13 weeks | | | |
| 40 °C /75% RH | 4 weeks | Acceptable | Acceptable | Acceptable |
| | 8 weeks | | | |
| | 13 weeks | | | |
| 4 °C | 4 weeks | Acceptable | Acceptable | Acceptable |
| | 8 weeks | | | |
| | 13 weeks | | | |
| Natural Sunlight / UV | 4 weeks | Acceptable | Acceptable | Acceptable |
| Freeze/Thaw (-10 °C, -30 °C, and room temperature) | 7 days | Acceptable | Acceptable | Acceptable |

**Table 7**

| **Stability Data** - **Composition** 3 | | | | |
|---|---|---|---|---|
| Conditions | Time points | **Color** | **Odor** | **Appearance** |
| 25 °C / 60% RH | 4 weeks | Acceptable | Acceptable | Acceptable |
| | 8 weeks | | | |
| | 13 weeks | | | |
| 40 °C /75% RH | 4 weeks | Acceptable | Acceptable | Acceptable |
| | 8 weeks | | | |
| | 13 weeks | | | |
| 4 °C | 4 weeks | Acceptable | Acceptable | Acceptable |
| | 8 weeks | | | |
| | 13 weeks | | | |
| Natural Sunlight / UV | 4 weeks | Acceptable | Acceptable | Acceptable |
| Freeze/Thaw (-10 °C, -30 °C, and room temperature) | 7 days | Acceptable | Acceptable | Acceptable |

The compositions of Tables 1-3 were loaded into roll-on dispensers and applied to surfaces to measure viscosity and delivery. The compositions of Tables 1-3 also had a comparable delivery through roll-on dispensers as commercial counterparts, each composition having a viscosity between 1000-4000 mPas.

In some embodiments, the antiperspirant and/or deodorant compositions of the present disclosure have a pearlescent appearance as evaluated by a panel of people. In particular, as illustrated in Table 8, a panel of 9 people was used to evaluate the pearlescent appearance of antiperspirant and/or deodorant compositions of the present disclosure using a combination of high HLB and low HLB surfactants as the pearling agent. The compositions of Table 8 are similar to those of Tables 1-2 but used different amounts of Steareth-2 and Steareth-21 as the pearling agent.

**Table 8**

| | | | Pearlescent Appearance? | | |
|---|---|---|---|---|---|
| | Steareth-2 | Steareth-21 | Yes | Maybe | No |
| Composition 4 | 25 % | 75 % | 9 | - | - |
| Composition 5* | 0 % | 100 % | - | 1 | 8 |
| Composition 6 | 66.6 % | 33.3 % | 9 | - | - |
| Composition 7 | 50 % | 50 % | 7 | - | -2 |
| Composition 8 | 33.3 % | 66.6 % | 7 | - | 2 |
| Composition 9 | 75 % | 25 % | 8 | - | 1 |

| | | | | | |
|---|---|---|---|---|---|
| *Comparative example | | | | | |

As illustrated in Table 8, when including combinations of high HLB and low HLB surfactants per embodiments of the present disclosure, visual evaluation of the deodorant and/or antiperspirant compositions of Table 8 by a panel of people indicated they possessed a recognizable pearlescent appearance.

Table 9 illustrates a set of pearlescent deodorant and/or antiperspirant compositions created according to the present disclosure. As illustrated in Table 9, the pearling agent is used as the surfactant in the deodorant and/or antiperspirant compositions and provides a pearlescent appearance, even though the deodorant and/or antiperspirant composition includes additional ingredient often found in commercially available deodorant and/or antiperspirant compositions.

**Table 9**

| **Composition 10** | | |
|---|---|---|
| **Ingredients** | | **Weight %** |
| Potassium Aluminum Sulphate or Aluminium Chlorohydrate | Deodorant Active or Antiperspirant Active | 2.0 % or 8-12% |
| Humectants, Emollients, Antioxidants, and Absorbents | | 10.4% |
| Steareth -2 | Surfactant / Pearling Agent | 3.2 % |
| Steareth -21 | Surfactant / Pearling Agent | 1.5 % |
| Caprylyl Glycol / Phenoxy Ethanol | Preservative | 1.1 % |
| Water | Carrier | Q. S. |
| **Total** | | **100 %** |

The deodorant and/or antiperspirant compositions created according to Table 9 also displayed comparable stability, irritability, and viscosity characteristics to those of commercial counterparts. In addition, a visual evaluation of the deodorant and/or antiperspirant compositions of Table 9 indicated they possessed a recognizable pearlescent appearance due to the combination of high HLB and low HLB surfactants used as the pearling agent.

Table 10 illustrates a pearlescent shower gel composition according to the present disclosure. As illustrated in Table 10, the pearling agent is used together with additional surfactants and/or emulsifiers in the shower gel composition, but still provides a pearlescent appearance.

**Table 10**

| **Composition 11** | | |
|---|---|---|
| **Ingredient** | | **Weight %** |
| Steareth -2 | Surfactant / Pearling Agent | 2.5 % |
| Steareth -21 | Surfactant / Pearling Agent | 1.5 % |
| SLES and Additional Surfactants | Surfactants | 20.7 % |
| Lactic Acid and Sodium Lactate | pH modifiers | 2.35% |
| Sodium Benzoate and Sodium Salicylate | Preservatives | 0.7 % |
| Water | Carrier | Q. S. |
| **Total** | | **100 %** |

The shower gel composition of Table 10 was prepared as follows: the formula amounts of the water, preservatives, and sodium lactate were mixed together and heated to 50 °C. The formula amounts of SLES was then mixed in. The formula amounts of Steareth -2 and Steareth - 21 were mixed together and heated to 50-80 °C, and the resulting mixture was then added to the mixture of water, preservatives, sodium lactate and SLES. The additional surfactants and the formula amounts of lactic acid was then added. Brine was added as needed.

The shower gel composition of Table 10 displayed comparable stability, irritability, and viscosity characteristics to those of commercial counterparts. In addition, a visual evaluation of the shower gel composition of Table 10 indicated it possessed a recognizable pearlescent appearance due to the combination of high HLB and low HLB surfactants used as a pearling agent.

Table 11 illustrates a shower gel compositions with similar components as that of Table 10, but lacking the pearling agent according to the present disclosure.

**Table 11 (comparative)**

| **Composition 12** | | |
|---|---|---|
| **Ingredient** | | **Weight %** |
| Humectants, Emollients, and Antimicrobials | Humectant | 7.30 % |
| SLES and Additional Surfactants | Surfactants | 20.7 % |
| Lactic Acid and Sodium Lactate | pH modifiers | 2.35% |
| Sodium Benzoate and Sodium Salicylate | Preservatives | 0.7 % |
| Water | Carrier | QS % |
| **Total** | | **100 %** |

A visual evaluation of the shower gel composition of Table 11 indicated it did not have a pearlescent appearance.

## Claims

1. Use of a pearling agent to impart a pearlescent or pearlescence-like appearance to cosmetic and personal care compositions, wherein the pearling agent is a surfactant combination of
one or more low HLB surfactants having an HLB value from 3 to 6, in an amount from 2 weight % to 5 weight % based on the total weight of the cosmetic or personal care composition; and
one or more high HLB surfactants having an HLB value from 12 to 16, in an amount from 1.5 weight % to 4.5 weight % based on the total weight of the cosmetic or personal care composition.

2. The use of a pearling agent according to claim 1, wherein the pearling agent is the only surfactant in the cosmetic or personal care composition.

3. The use of a pearling agent according to claim 1 or 2, wherein the cosmetic or personal care composition does not include additional pearling agents.

4. The use of a pearling agent according to any of claims 1-3, wherein the surfactant combination consists of Steareth-2 and Steareth-21.

## Patentansprüche

1. Verwendung eines Perlglanzmittels, um Kosmetik- oder Körperpflegezusammensetzungen ein perlglänzendes oder perlglanzähnliches Aussehen zu verleihen, wobei das Perlglanzmittel eine Tensidkombination ist aus
einem oder mehreren Tensiden mit niedrigem HLB-Wert von 3 bis 6 in einer Menge von 2 Gew.-% bis 5 Gew.-%, bezogen auf das Gesamtgewicht der Kosmetik- oder Körperpflegezusammensetzung; und
einem oder mehreren Tensiden mit hohem HLB-Wert von 12 bis 16 in einer Menge von 1,5 Gew.-% bis 4,5 Gew.-%, bezogen auf das Gesamtgewicht der Kosmetik- oder Körperpflegezusammensetzung.

2. Verwendung eines Perlglanzmittels nach Anspruch 1, wobei das Perlglanzmittel das einzige Tensid in der Kosmetik- oder Körperpflegezubereitung ist.

3. Verwendung eines Perlglanzmittels nach Anspruch 1 oder 2, wobei die Kosmetik- oder Körperpflegezusammensetzung keine weiteren Perlglanzmittel enthält.

4. Verwendung eines Perlglanzmittels nach einem der Ansprüche 1 bis 3, wobei die Tensidkombination aus Steareth-2 und Steareth-21 besteht.

## Revendications

1. Utilisation d'un agent nacrant pour conférer un aspect nacré ou semblable à de la nacre à des compositions cosmétiques et de soins personnels, dans laquelle l'agent nacrant est une combinaison de tensioactifs constituée :
d'un ou plusieurs tensioactifs à faible HLB ayant une valeur HLB comprise entre 3 et 6, en une quantité allant de 2 % en poids à 5 % en poids par rapport au poids total de la composition cosmétique ou de soin personnel ; et
d'un ou plusieurs tensioactifs à HLB élevé ayant une valeur HLB comprise entre 12 et 16, en une quantité allant de 1,5 % en poids à 4,5 % en poids par rapport au poids total de la composition cosmétique ou de soin personnel.

2. Utilisation d'un agent nacrant selon la revendication 1, dans laquelle l'agent nacrant est le seul tensioactif présent dans la composition cosmétique ou de soin personnel.

3. Utilisation d'un agent nacrant selon la revendication 1 ou 2, dans laquelle la composition cosmétique ou de soin personnel ne comprend pas d'agents nacrants supplémentaires.

4. Utilisation d'un agent nacrant selon l'une quelconque des revendications 1 à 3, dans laquelle la combinaison de tensioactifs est constituée de Steareth-2 et de Steareth-21.
